(19) Europäisches Patentamt
European Patent Office
Office européen des brevets

(11) Veröffentlichungsnummer : **0 036 991**
**B1**

(12) # EUROPÄISCHE PATENTSCHRIFT

(45) Veröffentlichungstag der Patentschrift : **16.11.83**

(21) Anmeldenummer : **81101913.2**

(22) Anmeldetag : **14.03.81**

(51) Int. Cl.³ : **C 07 C153/07**, C 07 D333/18, C 07 D307/38, C 07 D239/26, A 61 K 31/265, A 61 K 31/33

(54) **S-Alkyl-3-alkylthiopropionthioate, Verfahren zu ihrer Herstellung und ihre Verwendung in Arzneimitteln.**

(30) Priorität : **28.03.80 DE 3012000**

(43) Veröffentlichungstag der Anmeldung : **07.10.81 Patentblatt 81/40**

(45) Bekanntmachung des Hinweises auf die Patenterteilung : **16.11.83 Patentblatt 83/46**

(84) Benannte Vertragsstaaten : **AT BE CH DE FR GB IT LI NL**

(56) Entgegenhaltungen :
**US A 3 558 640**

**JOURNAL OF THE CHEMICAL SOCIETY, (B), 1971, Seiten 565-566 The Chemical Society of London, G.B. B. WLADISLAW et al. : « Interaction between the carbonyl group and an alpha-sulphur atom. Part I. Infrared and ultraviolet spectra of some alpha-(alkylthio)thioesters »**

**Die Akte enthält technische Angaben, die nach dem Eingang der Anmeldung eingereicht wurden und die nicht in dieser Patentschrift enthalten sind.**

(73) Patentinhaber : **Troponwerke GmbH & Co. KG**
**Berliner Strasse 156**
**D-5000 Köln 80 (DE)**

(72) Erfinder : **Jacobi, Haireddin, Dr.**
**Finkenweg 2**
**D-5672 Leichlingen (DE)**
Erfinder : **Opitz, Wolfgang, Dr.**
**Holzbachtalstrasse 60**
**D-5063 Overath (DE)**

(74) Vertreter : **Dill, Erwin, Dr. et al**
**c/o BAYER AG Zentralbereich Patente Marken und Lizenzen Bayerwerk**
**D-5090 Leverkusen 1 (DE)**

**EP 0 036 991 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

S-Alkyl-3-alkylthiopropionthioate, Verfahren zu ihrer Herstellung und ihre Verwendung in Arzneimitteln

Die vorliegende Erfindung betrifft neue S-Alkyl-3-alkylthiopropionthioate, Verfahren zu ihrer Herstellung sowie ihre Verwendung in Arzneimitteln, insbesondere in Arzneimitteln zur Behandlung entzündlicher Prozesse.

Die pharmazeutische Verwendung ähnlicher Verbindungen ist bereits in der DE-OS 2 753 768 und in der DE-OS 2 824 386 beschrieben. Die Verwertbarkeit der dort beanspruchten unsubstituierten Verbindungen ist jedoch stark eingeschränkt, da sie sehr geruchsintensiv sind. In J. Chem. Soc. (B) 1971, S. 565, Table I, ist in Beispiel XI S-Benzyl-3-(benzylthio)-thiopropionat genannt. Es ist jedoch keine Verwendung für diese Verbindung angegeben.

Ferner sind aus US-A 3 558 640 Propionsäureester mit entzündungshemmender Wirkung bekannt. Diese bekannten Ester enthalten in ihrem Esterteil Sauerstoff und sind nicht so wirksam wie die erfindungsgemäßen schwefelhaltigen Ester.

Die Erfindung betrifft neue S-Alkyl-3-alkylthiopropionthioate der allgemeinen Formel (I)

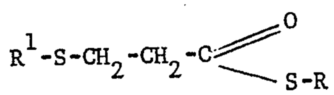
(I)

in welcher

R und $R^1$ gleich oder verschieden sind und jeweils für einen substituierten geradkettigen oder verzweigten Alkylrest mit 1 bis 10 Kohlenstoffatomen stehen, wobei der Alkylrest substituiert ist durch Amino, Phenyl, Naphthyl, Phenoxy, Naphthoxy, Alkylamino, Dialkylamino mit jeweils 1 bis 4 Kohlenstoffatomen in den Alkylgruppen oder durch Heteroaryl mit einem 5- bis 7-gliedrigen Ring, der als Heteroatom ein Sauerstoff-, Schwefel- oder Stickstoffatom enthält, wobei die genannten Phenyl-, Phenoxy- und Heteroarylreste ihrerseits gegebenenfalls durch 1, 2 oder 3 gleiche oder verschiedene Substituenten aus der Gruppe Halogen, Trifluormethyl, Nitro, Phenoxy, Benzyloxy, Alkyl oder Alkoxy mit je 1 bis 4 Kohlenstoffatomen substituiert sind, oder für einen mono-, di-, tri- oder tetracyclischen Cycloalkylrest mit 5 bis 10 Kohlenstoffatomen stehen, jedoch außer S-Benzyl-3-(benzylthio)-thiopropionat, $Ph—CH_2—S(CH_2)_2—CO—S—CH_2—Ph$.

Der in der Definition von R und $R^1$ genannte geradkettige oder verzweigte Alkylrest steht vorzugsweise für einen substituierten Alkylrest mit bis 6 Kohlenstoffatomen. Halogen steht vorzugsweise für Chlor oder Brom. Von besonderem Interesse sind Verbindungen der allgemeinen Formel (I), in welcher

R und $R^1$ gleich oder verschieden sind und jeweils für einen substituierten geradkettigen oder verzweigten Alkylrest mit 1 bis 6 Kohlenstoffatomen, wobei der Alkylrest substituiert ist durch Amino, Alkylamino, Dialkylamino, mit je 1 bis 4 Kohlenstoffatomen in den Alkylresten, durch Phenyl, Naphthyl, Phenoxy oder 5- bzw. 6-gliedriges Heteroaryl, das als Heteroatom ein Sauerstoff-, Schwefel- oder Stickstoffatom enthält, wobei die genannten Phenyl- und Phenoxyreste gegebenenfalls durch 1 oder 2 gleiche oder verschiedene Substituenten aus der Gruppe Halogen, Trifluormethyl, Nitro, Benzyloxy, Alkyl mit 1 bis 2 Kohlenstoffatomen oder Alkoxy mit 1 bis 2 Kohlenstoffatomen substituiert sind oder für einen Adamantylrest stehen.

Ein weiterer Gegenstand der vorliegenden Erfindung sind Verbindungen der allgemeinen Formel (I)

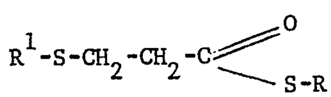
(I)

in welcher

R und $R^1$ gleich oder verschieden sind und jeweils für einen substituierten geradkettigen oder verzweigten Alkylrest mit 1 bis 10 Kohlenstoffatomen stehen, wobei der Alkylrest substituiert ist durch Amino, Phenyl, Naphthyl, Phenoxy, Naphthoxy, Alkylamino, Dialkylamino mit jeweils 1 bis 4 Kohlenstoffatomen in den Alkylgruppen oder durch Heteroaryl mit einem 5- bis 7-gliedrigen Ring, der als Heteroatom ein Sauerstoff-, Schwefel- oder Stickstoffatom enthält, wobei die genannten Phenyl-, Phenoxy- und Heteroarylreste ihrerseits gegebenenfalls durch 1, 2 oder 3 gleiche oder verschiedene Substituenten aus der Gruppe Halogen, Trifluormethyl, Nitro, Phenoxy, Benzyloxy, Alkyl oder Alkoxy mit je 1 bis 4 Kohlenstoffatomen substituiert sind, oder für einen mono-, di-, tri- oder tetracyclischen Cycloalkylrest mit 5 bis 10 Kohlenstoffatomen stehen, zur Verwendung bei der Bekämpfung von entzündlichen Prozessen.

Die erfindungsgemäßen Verbindungen der allgemeinen Formel (I) erhält man, indem man

a) Acrylsäurederivate der allgemeinen Formel (II)

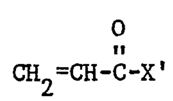
(II)

in welcher

X' Halogen oder Hydroxy bedeuten, mit der doppelt äquivalenten Menge eines Mercaptans der allgemeinen Formel (IIIa)

$$R—SH \qquad (IIIa)$$

in Gegenwart eines basischen Katalysators, bei Temperaturen zwischen 0 und 40 °C umsetzt oder

b) Thioether der allgemeinen Formel (IV)

$$RS-CH_2-CH_2-\overset{O}{\overset{\|}{C}}-X \qquad (IV)$$

in welcher

X für Hydroxyl, Halogen, Acyloxy oder einen Esterrest der Formel OR' steht, wobei R' gegebenenfalls substituiertes Alkyl, Aryl oder Aralkyl bedeutet, gegebenenfalls in Gegenwart eines Kondensationsmittels mit einem Mercaptan der allgemeinen Formel

$$(IIIa) \quad R—SH \quad \text{oder} \quad (IIIb) \quad R^1—SH$$

bei Temperaturen zwischen 0 und 40 °C umsetzt, wobei R und $R^1$ die oben angegebene Bedeutung haben, jedoch voneinander verschieden sind.

Bei Anwendung geeigneter Reaktionsbedingungen lassen sich durch Verwendung nur eines oder zweier verschiedener Mercaptane erfindungsgemäße Verbindungen der allgemeinen Formel I herstellen, in denen die Reste R und $R^1$ gleich oder voneinander verschieden sind.

Für die Herstellung von erfindungsgemäßen Verbindungen mit identischen Mercaptanresten ($R = R^1$) besteht eine bevorzugte Verfahrensvariante darin, daß die Acrylsäurederivate der allgemeinen Formel (II), in welcher X für Halogen, insbesondere Chlor, steht, mit der doppelt äquivalenten Menge eines Mercaptans der allgemeinen Formel (III) in Gegenwart eines Äquivalents Aklalilauge direkt umsetzt.

Als inerte organische Lösungsmittel seien vorzugsweise genannt cyclische Ether wie Dioxan oder Tetrahydrofuran, Ketone wie z. B. Aceton oder Dimethylformamid.

Als Aktivierungsmittel bzw. Kondensationsmittel seien vorzugsweise genannt Dicyclohexylcarbodiimid, Carbonyldiimidazol, Thionylchlorid oder Phosphorsäurehalogenide (vgl. Angew. Chem. *74*, 407 (1962) und J. Amer. Chem. Soc. *77*, 1067 (1955)).

Als basische Katalysatoren seien vorzugsweise genannt, Alkalihydroxide.

Als Alkalihydroxide seien vorzugsweise genannt Natriumhydroxid und Kaliumhydroxid, insbesondere Natriumhydroxid, wobei vorzugsweise maximal äquimolare Mengen bezogen auf die Verbindungen der allgemeinen Formel (II), eingesetzt werden.

Die erfindungsgemäß einsetzbaren Acrylsäurederivate der allgemeinen Formel (II) sind bekannt oder können nach bekannten Methoden hergestellt werden (vgl. Beilstein E III *2*, 1233 und J. Org. Chem. *9*, 506 (1944)).

Die erfindungsgemäß verwendeten Mercaptane der allgemeinen Formel (III) sind bekannt oder können nach bekannten Methoden hergestellt werden (z. B. Org. Synth. Coll. Vol. *3*, 363).

Beispielhaft seien folgende Mercaptane genannt :

2-Fluorbenzylmercaptan,

2,6-Dimethoxybenzylmercaptan, F = 78,5-80,5 °C,

3,4,5-Trimethoxybenzylmercaptan, wachsige Substanz $^1H$-NMR ($CDCl_3$), δppm = 1.75 (T, 1H) ; 3.62 (D, 2H) ; 3.78 (S, 9H) ; 6.45 (S, 2H).

Überraschenderweise zeigen die erfindungsgemäßen Verbindungen eine vorteilhafte antiphlogistische Wirkung bei sehr guter Verträglichkeit. Darüber hinaus ist die Geruchsintensität der erfindungsgemäßen Schwefelverbindungen wesentlich geringer als bei den aus dem Stand der Technik bekannten Schwefelverbindungen. Sie können mit geringerer Geruchsbelästigung verarbeitet und angewendet werden und stellen somit eine Bereicherung der Pharmazie dar.

Zur vorliegenden Erfindung gehören pharmazeutische Zubereitungen, die neben nichttoxischen, inerten, pharmazeutisch geeigneten Trägerstoffen einen oder mehrere erfindungsgemäße Wirkstoffe enthalten, oder die aus einem oder mehreren erfindungsgemäßen Wirkstoffen bestehen, sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z. B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z. B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einder Applikation verabreicht wird und die gewöhnlich einer ganzen, einer halben oder einem Drittel oder einem Viertel einer Tagesdosis entspricht.

Unter nichttoxischen, inerten pharmazeutisch geeigneten Trägerstoffen sind feste, halbfeste oder

flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben üblichen Trägerstoffen enthalten, wie Füll- und Streckmittel (z. B. Stärken, Milchzucker, Rohrzucker, Glukose, Mannit und Kieselsäure), Bindemittel (z. B. Carboxymethylcellulose, Alginate, Gelatine, Polyvinylpyrrolidon), Feuchthaltemittel (z. B. Glycerin), Sprengmittel (z. B. Agar-Agar, Calciumcarbonat und Natriumbicarbonat), Lösungsverzögerer (z. B. Paraffin) und Resorptionsbeschleuniger (z. B. quaternäre Ammoniumverbindungen), Adsorptionsmittel (z. B. Kaolin und Bentonit) und Gleitmittel (z. B. Talkum, Calcium- und Magnesiumstearat und feste Polyethylenglykole) oder Gemische der aufgeführten Stoffe.

Die Tabletten, Dragees, Kapseln, Pillen und Granulate können mit den üblichen, gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes, gegebenenfalls verzögert, abgeben, wobei als Einbettungsmassen z. B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen zur Erzielung eines Retardeffektes.

Suppositorien können neben dem oder den Wirkstoffen die üblichen wasserlöslichen oder wasserunlöslichen Trägerstoffe enthalten, z. B. Polyethylenglykole, Fette, z. B. Kakaofett und höhere Ester (z. B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten (z. B. tierische und pflanzliche Fette, Wachse, Paraffine, Stärken, Tragant) oder Gemische dieser Stoffe.

Puder und Sprays können neben dem oder den Wirkstoffen die üblichen Trägerstoffe enthalten (z. B. Milchzucker, Talkum, Kieselsäure, Aluminiumhydroxid) oder Gemische dieser Stoffe. Sprays können zusätzlich die üblichen Treibmittel enthalten.

Lösungen und Emulsionen können neben dem oder den Wirkstoffen die üblichen Trägerstoffe wie Lösungsmittel, Lösungsvermittler und Emulgatoren z. B. Wasser, Ethylalkohol, Isopropylalkohol, Ethylcarbonat, Ethylacetat, Benzylalkohol, Benzylbenzoat, Propylenglykol, Öle, insbesondere Baumwollsaatöl, Erdnußöl, Maiskeimöl, Olivenöl und Sesamöl, Glycerin, Polyethylenglykole oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel (z. B. Wasser, Ethylalkohol, Propylenglykol), Suspendiermittel (z. B. ethoxylierte Isostearylalkohole, Polyoxyethylensorbit- und Sorbitanester, mikrokristalline Cellulose) oder Gemische dieser Stoffe enthalten.

Die genannten Formulierungsformen können auch Färbemittel, Konservierungsstoffe sowie geruchs- und geschmacksverbessernde Zusätze, z. B. Pfefferminzöl und Eukalyptusöl und Süßmittel, z. B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung, vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z. B. durch Mischen des oder der Wirkstoffe mit dem oder den Trägerstoffen.

Zur vorliegenden Erfindung gehört auch die Verwendung von pharmazeutischen Zubereitungen, die einen oder mehrere erfindungsgemäße Wirkstoffe enthalten, in der Human- und Veterinärmedizin zur Verhütung, Besserung und/oder Heilung entzündlicher Prozesse in menschlichen und tierischen Organismus.

Die Wirkstoffe oder die pharmazeutischen Zubereitungen können lokal, oral, parenteral, intraperitoneal und/oder rectal, vorzugsweise parenteral, appliziert werden.

Im allgemeinen ist es in der Humanmedizin als vorteilhaft anzusehen, den oder die erfindungsgemäßen Wirkstoffe in Gesamtmengen von etwa 20 bis 800, vorzugsweise 100 bis 500 mg je 24 Stunden, gegebenenfalls in Form mehrerer Einzelgaben zur Erzielung der gewünschten Ergebnisse, zu verabreichen. Eine Einzelgabe enthält den oder die erfindungsgemäßen Wirkstoffe, vorzugsweise in Mengen von etwa 10 bis etwa 300, insbesondere 50 bis 200 mg/Dosis. Es kann jedoch erforderlich sein, von den genannten Dosierungen abzuweichen und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben angeführte Wirkstoffmenge überschritten werden muß.

Die Festlegung der jeweils erforderlichen optimalen Dosierung und Applikationsart der Wirkstoffe kann durch jeden Fachmann aufgrund seines Fachwissens leicht erfolgen.

## Ausführungsbeispiele

### Beispiel 1

S-(3,4,5-Trimethoxybenzyl)-3-(3,4,5-trimethoxybenzylthio)propionthioat

Ein Gemisch aus 4,28 g (20 mMol) 3,4,5-Trimethoxybenzylmercaptan und 8 ml 5 %iger Natronlauge wurde bei Raumtemperatur portionsweise mit 0,81 ml (10 mMol) Acrylsäurechlorid versetzt und bis zur Beendigung der exothermen Reaktion gerührt. Nach Verdünnen mit Wasser wurde das Reaktionsgemisch mit Ether extrahiert, die Etherphase eingedampft und der Rückstand an einer Kieselgelsäule aus Ether/Diisopropylether (1 : 1) chromatographiert. Schmelzpunkt 88 bis 89 °C. Ausbeute 1,55 g (32,1 % der Theorie).

### Beispiel 2

S-[2-(Diisopropylamino)ethyl]-3-[2-(diisopropylamino)-ethylthio] propionthioat

Eine auf 0 °C gekühlte Lösung von 1,8 g (25 mMol) Acrylsäure und 8,07 g (50 mmol) 2-(Diisopropylamino)ethylmercaptan (durch Extraktion einer alkalisch gestellten Lösung des Hydrochlorids mit Dichlormethan erhalten) wurde mit 5,15 g (25 mMol) Dicyclohexylcarbodiimid versetzt und 5 Stunden bei Raumtemperatur gerührt und anschließend über Nacht stehen gelassen. Nach Abfiltrieren des ausgefallenen Dicyclohexylharnstoffes wurde das Filtrat im Vakuum eingedampft und das resultierende Öl zweimal im Kugelrohr destilliert. $Kp_{6,65\ Pa}$ = 200 °C, Ausbeute 7,1 g (75,4 % der Theorie).

### Beispiel 3

S-(4-Fluorbenzyl)-3-benzylthiopropionthioat

Eine auf 0 °C gekühlte Lösung von 5,9 g (30 mMol) 3-Benzylthiopropionsäure und 4,26 g (30 mmol) 4-Fluorbenzylmercaptan in 25 ml Dichlormethan wurde mit 6,18 g (30 mMol) Dicyclohexylcarbodiimid in 20 ml Dichlormethan versetzt und über Nacht bei Raumtemperatur aufbewahrt. Nach Abfiltrieren des Dicyclohexylharnstoffes und Abziehen des Lösungsmittels wurde das Rohprodukt zunächst durch Chromatographie an Kieselgel aus Dichlormethan/Petrolether 1 : 2 und anschließend durch Destillation im Kugelrohr gereinigt.

$Kp_{6,65\ Pa}$ = 215 bis 240 °C

Ausbeute 7,4 g (77 % der Theorie).

$^1H$-NMR ($CDCl_3$) : δ ppm = 2.65 (S, 4H) ; 3.60 (S, 2H) ; 3.97 (S, 2H) ; 6.90 (M, 4H) ; 7.25 (S, 5H).

### Beispiel 4

Analog zu Beispiel 3 wurde erhalten :

S-(Furyl-2-methylthio)-3-benzylthiopropionthioat

$Kp_{6,65\ Pa}$ = 210 bis 220 °C

Ausbeute 73,6 %.

$^1H$-NMR ($CDCl_3$) : δ ppm = 2.64 (S, 4H) ; 3.58 (S, 2H) ; 4.02 (S, 2H) ; 6.09 (M, 2H) ; 7.14 (breites S, 6H).

Die in der nachstehenden Tabelle aufgeführten Verbindungen Nr. 5 bis 30 werden analog Beispiel 1 und die Verbindungen Nr. 31 bis 34 analog Beispiel 2 hergestellt.

(Siehe Tabellen Seite 6 ff.)

| Beispiel-Nr. | Verbindung | Ausbeute % d. Th. | Kp/F | $^1$H-NMR ($CDCl_3$), 60 MHz, Perkin Elmer R 24 A, $\delta$ ppm (TMS) |
|---|---|---|---|---|
| 5 | S-(3-Trifluormethylbenzyl)-3-(3-trifluormethylbenzylthio)propionthioat | 40 | Öl | 2.71 (S, 4H); 3.68 (S, 2H); 4.08 (S, 2H); 7.25 - 7.62 (M, 8H) |
| 6 | S-(3,4-Dichlorbenzyl)-3-(3,4-dichlorbenzylthio)propionthioat | 58 | Öl | 2.68 (S, 4H); 3.58 (S, 2H); 3.98 (S, 2H); 6.57 - 7.36 (M, 6H) |
| 7 | S-(4-Methoxybenzyl)-3-(4-methoxybenzylthio)propionthioat | 29,7 | F= 41-42°C | 2.62 (S, 4H); 3.55 (S, 2H); 3.63 (S, 6H); 3.97 (S, 2H); 6.90 (Q, 8H) |
| 8 | S-(3-Methylbenzyl)-3-(3-methylbenzylthio)propionthioat | 18,2 | Öl | 2.24 (S, 6H); 2.68 (S, 4H); 3.60 (S, 2H); 4.01 (S, 2H); 7.02 (S, 8H) |
| 9 | S-(Furyl-2-methyl)-3-(furyl-2-methylthio)propionthioat | 23 | Öl | 2.72 (S, 4H); 3.65 (S, 2H); 4.10 (S, 2H); 6.05 - 6.3 (M, 4H); 7.15 - 7.32 (M, 2H) |
| 10 | S-(4-Chlorbenzyl)-3-(4-chlorbenzylthio)propionthioat | 60,6 | F= 41-42°C | 2.65 (S, 4H); 3.55 (S, 2H); 3.97 (2H); 7.12 (S, 8H) |
| 11 | S-(2,6-Dichlorbenzyl)-3-(2,6-dichlorbenzylthio)propionthioat | 38,6 | Öl | 2.85 (S, 4H); 3.98 (S, 2H); 4.41 (S, 2H); 6.8 - 7.3 (M, 6H) |
| 12 | S-(3,4,5-Trimethoxybenzyl)-3-(3,4,5-trimethoxybenzylthio)propionthioat | 32,1 | F= 88-89°C | 2.73 (S, 4H); 3.61 (S, 2H); 3.76 (S, 18H); 4.0 (S, 2H); 6.41 (S, 2H); 6.45 (S, 2H) |
| 13 | S-(4-Fluorbenzyl)-3-(4-fluorbenzylthio)propionthioat | 74,9 | Öl | 2.69 (S, 4H); 3.58 (S, 2H); 3.99 (S, 2H); 6.6 - 7.3 (M, 8H) |
| 14 | S-(Diphenylmethyl)-3-(diphenylmethylthio)propionthioat | 14,9 | Öl | 2.61 (S, 4H); 5.01 (S, 1H); 5.83 (S, 1H); 6.95 - 7.4 (M, 20H) |

(Fortsetzung)

| Beispiel-Nr. | Verbindung | Ausbeute % d. Th. | Kp/F | $^1$H-NMR ($CDCl_3$), 60 MHz, Perkin Elmer R 24 A, δ ppm (TMS) |
|---|---|---|---|---|
| 15 | S-(Pyridyl-2-methyl)-3-(pyridyl-2-methylthio)propionthioat | 49,4 | Öl | 2.5 - 3,4 (M, 8H); 6.5 - 8.0 (M, 6H); 8.3 - 8.5 (M, 2H) |
| 16 | S-(2,6-Dimethoxybenzyl)-3-(2,6-dimethoxybenzylthio)propionthioat | 18,1 | F= 81-83°C | 2,73 (S, 4H); 3.71 (S, 14H); 4.15 (S, 2H); 6.4 (D, 4H); 6.82 - 7.18 (M, 2H) |
| 17 | S-(1-Phenylpropyl)-3-(1-phenylpropylthio)propionthioat | 7 | Öl | 0.85 (T, 6H); 1.45-2.1 (M, 4H); 2.51 (S, 4H); 3.52 - 3.68 (T, 1H); 4.38 (T, 1H); 7.15 (S, 10H) |
| 18 | S-(Thienyl-2-methyl)-3-(thienyl-2-methylthio)propionthioat | 44,5 | Öl | 2.71 (S, 4H); 3.81 (S, 2H); 4.22 (S, 2H); 6.50 - 7.32 (M, 6H) |
| 19 | S-(1-Phenylethyl)-3-(1-phenylethylthio)propionthioat | 22,7 | $Kp_{4,65Pa}$ = 250°C (Kugelrohr) | 1.6 (T, 6H); 2.55 (S, 4H); 3.85 (Q, 1H); 4.65 (Q, 1H); 7.28 (S, 10H) |
| 20 | S-(4-Nitrobenzyl)-3-(4-nitrobenzylthio)propionthioat | 24,2 | F= 60-61°C | 2.72 (S, 4H); 3.71 (S, 2H); 4.11 (S, 2H); 7.16 - 8.2 (M, 8H) |
| 21 | S-(2-Trifluormethylbenzyl)-3-(2-trifluormethylbenzylthio)propionthioat | 47,5 | $Kp_{4,65 Pa}$ =240-50°C (Kugelrohr) | 2,7 (S, 4H); 3.66 (S, 2H); 4.07 (S, 2H); 7.2 - 7.55 (M, 8H) |
| 22 | S-(4-Methylbenzyl)-3-(4-methylbenzylthio)propionthioat | 31,3 | $Kp_{4,65 Pa}$ =250°C (Kugelrohr) | 2.23 (S, 6H); 2.63 (S, 4H); 3.57 (S, 2H); 3.98 (S, 2H); 7.01 (S, 8H) |

(Fortsetzung)

| Beispiel-Nr. | Verbindung | Ausbeute % d. Th. | Kp/F | $^1H$-NMR ($CDCl_3$), 60 MHz, Perkin Elmer R 24 A, δ ppm (TMS) |
|---|---|---|---|---|
| 23 | S-(Naphthyl-2-methyl)-3-(naphthyl-2-methylthio)propionthioat | 42,3 | wachsige Substanz | 2.66 (S, 4H); 3.97 (S, 2H); 4.42 (S, 2H); 6.99 - 7.98 (M, 14H) |
| 24 | S-(4-Benzyloxybenzyl)-3-(4-benzyloxy-benzylthio)propionthioat | 38,8 | F= 96-98°C | 2.67 (S, 4H); 3.56 (S, 2H); 3.98 (S, 2H); 4.95 (S, 4H); 6.93 (Q, 8H); 7.23 (S, 10H) |
| 25 | S-(3-Fluorbenzyl)-3-(3-fluorbenzylthio)propionthioat | 23,1 | $Kp_{6,65\ Pa}$ =245-50° (Kugelrohr) | 2.67 (S, 4H); 3.56 (S, 2H); 3.98 (S, 2H); 6.6-7.35 (M, 8H) |
| 26 | S-(2-Fluorbenzyl)-3-(2-fluorbenzylthio)propionthioat | 40,4 | $Kp_{6,65\ Pa}$ =235° (Kugelrohr) | 2.70 (S, 4H); 3.64 (S, 2H); 4.05 (S, 2H); 6.65-7.35 (M, 8H) |
| 27 | S-(2-Phenylisopropyl)-3-(2-phenylisopropylthio)propionthioat | 36,3 | Öl | 1.1-1.35 (2D, 6H); 2.4-3.1 (M, 9H); 3.4-3.95 (M, 1H); 7.1 (S, 10H) |

(Fortsetzung)

| Beispiel-Nr. | Verbindung | Ausbeute % d. Th. | Kp/F | $^1$H-NMR ($CDCl_3$), 60 MHz, Perkin Elmer R 24 A, δ ppm (TMS) |
|---|---|---|---|---|
| 28 | S-(2-Phenoxyethyl)-3-(2-phenoxy-ethylthio)propionthioat | 18,4 | Kp 4,65 Pa = 240-50° (Kugelrohr) | 2.70-3.05 (M, 6H); 3.22 (T, 2H); 3.86-4.23 (M, 4H); 6.68-7.33 (M, 10H) |
| 29 | S-(2-Cyclohexylethyl)-3-(2-cyclo-hexylethylthio)propionthioat | 16,1 | Kp 4,65 Pa = 240-50° | 0.51-1.95 (M, 26H); 2.46 (T, 2H); 2.74 (S, 4H); 2.82 (T, 2H) |
| 30 | S-(Adamantyl-1)-3-(adamantyl-1-thio)propionthioat | 30,7 | F= 108-110° | 1.53-2.32 (M, 30H); 2.63 (S, 4H) |
| 31 | S-(2-Phenylethyl)-3-(2-phenyl-ethylthio)propionthioat | 34,3 | Kp 4,65 Pa = 250° (Kugelrohr) | 2.67 (S, 4H); 2.6-3.15 (M, 8H); 7.05 (S, 10H) |
| 32 | S-[2-(Dimethylamino)ethyl]-3-[2-(dimethylamino)ethylthio]-propionthioat | 30,4 | Kp 4,65 Pa =190-200° (Kugelrohr) | 1.72 (M, 4H); 2.18 (S, 12H); 2.2-2.5 (M 4H); 2.7-3.01 (M, 4H) |

(Fortsetzung)

| Beispiel-Nr. | Verbindung | Ausbeute % d. Th. | Kp/F | $^1H$-NMR ($CDCl_3$), 60 MHz, Perkin Elmer R 24 A, δ ppm (TMS) |
|---|---|---|---|---|
| 33 | S-[2-Diethylaminoethyl]-3-[2-(diethylamino)ethylthio]propionthioat | 38,7 | $Kp_{4,65 Pa}$ =165-70° (Kugelrohr) | 1.00 (T, 12H); 2.50 (Q, 8H); 2.58 (S, 4H); 2.60-3.02 (M, 4H) |
| 34 | S-[2-(Diisopropylamino)ethyl]-3-[2-diisopropylamino)ethylthio]-propionthioat | 75,4 | $Kp_{6,65 Pa}$ 200° (Kugelrohr) | 1.01 (D, 24H); 2.50-3.20 (M, 16H) |

**Ansprüche**

1. S-Alkyl-3-alkylthiopropionthioate der allgemeinen Formel (I)

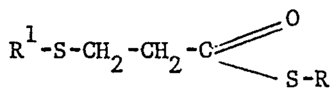

(I)

in welcher

R und $R^1$ gleich oder verschieden sind und jeweils für einen substituierten geradkettigen oder verzweigten Alkylrest mit 1 bis 10 Kohlenstoffatomen stehen, wobei der Alkylrest substituiert ist durch Amino, Phenyl, Naphthyl, Phenoxy, Naphthoxy, Alkylamino, Dialkylamino mit jeweils 1 bis 4 Kohlenstoffatomen in den Alkylgruppen oder durch Heteroaryl mit einem 5- bis 7-gliedrigen Ring, der als Heteroatom ein Sauerstoff-, Schwefel- oder Stickstoffatom enthält, wobei die genannten Phenyl-, Phenoxy- und Heteroarylreste ihrerseits gegebenenfalls durch 1, 2 oder 3 gleiche oder verschiedene Substituenten aus der Gruppe Halogen, Trifluormethyl, Nitro, Phenoxy, Benzyloxy, Alkyl oder Alkoxy mit je 1 bis 4 Kohlenstoffatomen substituiert sind, oder für einen mono-, di-, tri- oder tetracyclischen Cycloalkylrest mit 5 bis 10 Kohlenstoffatomen stehen, jedoch außer S-Benzyl-3-(benzylthio)-thiopropionat, $Ph—CH_2—S(CH_2)_2—CO—S—CH_2—Ph$.

2. S-Alkyl-3-alkylthiopropionthioate der allgemeinen Formel (I) gemäß Anspruch 1, in welcher

R und $R^1$ gleich oder verschieden sind und jeweils für einen substituierten geradkettigen oder verzweigten Alkylrest mit 1 bis 6 Kohlenstoffatomen, wobei der Alkylrest substituiert ist durch Amino, Alkylamino, Dialkylamino, mit je 1 bis 4 Kohlenstoffatomen in den Alkylresten, durch Phenyl, Naphthyl, Phenoxy oder 5- bzw. 6-gliedriges Heteroaryl, das als Heteroatom ein Sauerstoff-, Schwefel- oder Stickstoffatom enthält, wobei die genannten Phenyl- und Phenoxyreste gegebenenfalls durch 1 oder 2 gleiche oder verschiedene Substituenten aus der Gruppe Halogen, Trifluormethyl, Nitro, Benzyloxy, Alkyl mit 1 bis 2 Kohlenstoffatomen oder Alkoxy mit 1 bis 2 Kohlenstoffatomen substituiert sind, oder für einen Adamantylrest stehen.

3. S-(Adamantyl-1)-3-(adamantyl-1-thio)-propionthioat.

4. Verfahren zur Herstellung von Verbindungen der allgemeinen Formel (I) gemäß Anspruch 1 bis 3, dadurch gekennzeichnet, daß man

a) Acrylsäurederivate der allgemeinen Formel (II)

$$CH_2=CH-\overset{O}{\overset{\|}{C}}-X' \qquad (II)$$

in welcher

X' Halogen oder Hydroxy bedeuten, mit der doppelt äquivalenten Menge eines Mercaptans der allgemeinen Formel (IIIa)

$$R—SH \qquad (IIIa)$$

in Gegenwart eines basischen Katalysators, bei Temperaturen zwischen 0 und 40 °C umsetzt oder

b) Thioether der allgemeinen Formel (IV)

$$RS-CH_2-CH_2-\overset{O}{\overset{\|}{C}}-X \qquad (IV)$$

in welcher

X für Hydroxyl, Halogen, Acyloxy oder einen Esterrest der Formel OR' steht, wobei R' gegebenenfalls substituiertes Alkyl, Aryl oder Aralkyl bedeutet, gegebenenfalls in Gegenwart eines Kondensationsmittels mit einem Mercaptan der allgemeinen Formel

(IIIa) $R—SH$ oder (IIIb) $R^1—SH$

bei Temperaturen zwischen 0 und 40 °C umsetzt, wobei R und $R^1$ die oben angegebene Bedeutung haben, jedoch voneinander verschieden sind.

5. Verbindungen der allgemeinen Formel (I) gemäß Anspruch 1, zur Verwendung als Arzneimittel.

6. Verbindungen der allgemeinen Formel (I)

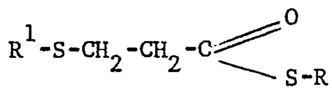

(I)

in welcher

R und $R^1$ gleich oder verschieden sind und jeweils für einen substituierten geradkettigen oder verzweigten Alkylrest mit 1 bis 10 Kohlenstoffatomen stehen, wobei der Alkylrest substituiert ist durch Amino, Phenyl, Naphthyl, Phenoxy, Naphthoxy, Alkylamino, Dialkylamino mit jeweils 1 bis 4 Kohlenstoffatomen in den Alkylgruppen oder durch Heteroaryl mit einem 5- bis 7-gliedrigen Ring, der als Heteroatom ein Sauerstoff-, Schwefel- oder Stickstoffatom enthält, wobei die genannten Phenyl-, Phenoxy- und Heteroarylreste ihrerseits gegebenenfalls durch 1, 2 oder 3 gleiche oder verschiedene Substituenten aus der Gruppe Halogen, Trifluormethyl, Nitro, Phenoxy, Benzyloxy, Alkyl oder Alkoxy mit je 1 bis 4 Kohlenstoffatomen substituiert sind, oder für einen mono-, di-, tri- oder tetracyclischen Cycloalkylrest mit 5 bis 10 Kohlenstoffatomen stehen, zur Verwendung bei der Bekämpfung von entzündlichen Prozessen.

7. Arzneimittel enthaltend mindestens eine Verbindung der allgemeinen Formel (I) gemäß Anspruch 1.

8. Antiphlogistisches Arzneimittel enthaltend mindestens eine Verbindung der allgemeinen Formel I

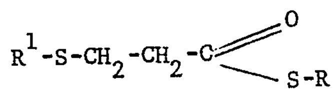

(I)

in welcher

R und $R^1$ gleich oder verschieden sind und jeweils für einen substituierten geradkettigen oder verzweigten Alkylrest mit 1 bis 10 Kohlenstoffatomen stehen, wobei der Alkylrest substituiert ist durch Amino, Phenyl, Naphthyl, Phenoxy, Naphthoxy, Alkylamino, Dialkylamino mit jeweils 1 bis 4 Kohlenstoffatomen in den Alkylgruppen oder durch Heteroaryl mit einem 5- bis 7-gliedrigen Ring, der als Heteroatom ein Sauerstoff-, Schwefel- oder Stickstoffatom enthält, wobei die genannten Phenyl-, Phenoxy- und Heteroarylreste ihrerseits gegebenenfalls durch 1, 2 oder 3 gleiche oder verschiedene Substituenten aus der Gruppe Halogen, Trifluormethyl, Nitro, Phenoxy, Benzyloxy, Alkyl oder Alkoxy mit je 1 bis 4 Kohlenstoffatomen substituiert sind, oder für einen mono-, di-, tri- oder tetracyclischen Cycloalkylrest mit 5 bis 10 Kohlenstoffatomen stehen.

9. Verfahren zur Herstellung von Arzneimitteln, dadurch gekennzeichnet, daß man Verbindungen der allgemeinen Formel (I) gemäß Anspruch 1 gegebenenfalls unter Verwendung inerter Hilfs- und Trägerstoffe in eine geeignete Applikationsform überführt.

**Claims**

1. S-Alkyl 3-alkylthiopropionothioates of the general formula (I)

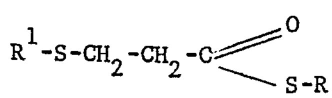

(I)

in which

R and $R^1$ are identical or different and each represent a substituted straight-chain or branched alkyl radical with 1 to 10 carbon atoms, the alkyl radical being substituted by amino, phenyl, naphthyl, phenoxy, naphthoxy or alkylamino or dialkylamino, each with 1 to 4 carbon atoms in the alkyl groups, or by heteroaryl with a 5-membered to 7-membered ring which contains an oxygen, sulphur or nitrogen atom as the hetero-atom, the said phenyl, phenoxy and heteroaryl radicals in turn being optionally substituted by 1, 2 or 3 identical or different substituents from the group comprising halogen, trifluoromethyl, nitro, phenoxy, benzyloxy and alkyl or alkoxy, each with 1 to 4 carbon atoms, or represent a mono-, di-, tri- or tetra-cyclic cycloalkyl radical with 5 to 10 carbon atoms, but with the exception of S-benzyl-3-(benzylthio)-thiopropionate, $Ph—CH_2—S(CH_2)_2—CO—S—CH_2—Ph$.

2. S-Alkyl 3-alkylthiopropionothioates of the general formula (I) according to Claim 1, in which

R and $R^1$ are identical or different and each represent a substituted straight-chain or branched alkyl radical with 1 to 6 carbon atoms, the alkyl radical being substituted by amino or alkylamino or dialkylamino, each with 1 to 4 carbon atoms in the alkyl radicals, or by phenyl, naphthyl, phenoxy or 5-membered to 6-membered hetero-aryl which contains an oxygen, sulphur or nitrogen atom as the heteroatom, the said phenyl and phenoxy radicals being optionally substituted by 1 or 2 identical or different substituents from the group comprising halogen, trifluoromethyl, nitro, benzyloxy or alkyl with 1 to 2 carbon atoms or alkoxy with 1 to 2 carbon atoms, or represent an adamantyl radical.

3. S-(Adamant-1-yl) 3-(adamantyl-1-thio)-propionothioate.

4. Process for the preparation of compounds of the general formula (I) according to Claim 1 to 3,

characterised in that

a) acrylic acid derivatives of the general formula (II)

$$CH_2=CH-\overset{O}{\overset{\|}{C}}-X' \qquad (II)$$

in which

X' denotes halogen or hydroxyl, are reacted with twice the equivalent amount of a mercaptan of the general formula (IIIa)

$$R—SH \qquad (IIIa)$$

in the presence of a basic catalyst, at temperatures between 0 and 40 °C, or

b) thio-ethers of the general formula (IV)

$$RS-CH_2-CH_2-\overset{O}{\overset{\|}{C}}-X \qquad (IV)$$

in which

X represents hydroxyl, halogen, acyloxy or an ester radical of the formula OR', wherein R' denotes optionally substituted alkyl, aryl or aralkyl, are reacted, optionally in the presence of a condensation agent, with a mercaptan of the general formula

(IIIa) R—SH or (IIIb) $R^1$—SH

wherein

R and $R^1$ have the meaning indicated above but are different from one another, at temperatures between 0 and 40 °C.

5. Compounds of the general formula (I) according to Claim 1, for use as medicaments.

6. Compounds of the general formula (I)

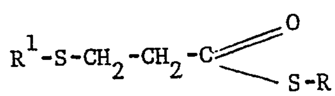

(I)

in which

R and $R^1$ are identical or different and each represent a substituted straight-chain or branched alkyl radical with 1 to 10 carbon atoms, the alkyl radical being substituted by amino, phenyl, naphthyl, phenoxy, naphthoxy or alkylamino or dialkylamino, each with 1 to 4 carbon atoms in the alkyl groups, or by hetero-aryl with a 5-membered to 7-membered ring which contains an oxygen, sulphur or nitrogen atom as the hetero-atom, the said phenyl, phenoxy and hetero-aryl radicals in turn being optionally substituted by 1, 2 or 3 identical or different substituents from the group comprising halogen, trifluoromethyl, nitro, phenoxy, benzyloxy and alkyl or alkoxy, each with 1 to 4 carbon atoms, or represent a mono-, di-, tri- or tetra-cyclic cycloalkyl radical with 5 to 10 carbon atoms, for use in combating inflammatory processes.

7. Medicaments containing at least one compound of the general formula (I) according to Claim 1.

8. Antiphlogistic medicament containing at least one compound of the general formula (I)

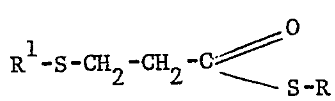

(I)

in which

R and $R^1$ are identical or different and each represent a substituted straight-chain or branched alkyl radical with 1 to 10 carbon atoms, the alkyl radical being substituted by amino, phenyl, naphthyl, phenoxy, naphthoxy or alkylamino or dialkylamino, each with 1 to 4 carbon atoms in the alkyl groups, or by hetero-aryl with a 5-membered to 7-membered ring which contains an oxygen, sulphur or nitrogen atom as the hetero-atom, the said phenyl, phenoxy and heteroaryl radicals in turn being optionally substituted by 1, 2 or 3 identical or different substituents from the group comprising halogen, trifluoromethyl, nitro, phenoxy, benzyloxy and alkyl or alkoxy, each with 1 to 4 carbon atoms, or represent a mono-, di-, tri- or tetra-cyclic cycloalkyl radical with 5 to 10 carbon atoms.

9. Process for the preparation of medicaments, characterised in that compounds of the general formula (I) according to Claim 1 are converted into a suitable form for administration, optionally using inert auxiliaries and excipients.

**Revendications**

1. 3-Alkylthiopropionothioates de S-alkyle de formule générale (I)

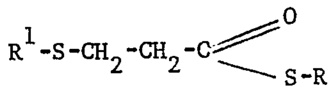

(I)

dans laquelle

R et $R^1$, identiques ou différents, représentent chacun un groupe alkyle substitué à chaîne droite ou ramifiée en $C_1$-$C_{10}$, le groupe alkyle étant substitué par des groupes amino, phényle, naphtyle, phénoxy, naphtoxy, alkylamino, dialkylamino contenant 1 à 4 atomes de carbone dans chacun des groupes alkyle, ou par un groupe hétéroaryle à cycle de 5 à 7 chaînons contenant en tant qu'hétéroatome un atome d'oxygène, de soufre ou d'azote, les groupes phényle, phénoxy et hétéroaryle mentionnés pouvant eux-mêmes porter éventuellement 1, 2 ou 3 substituants identiques ou différents pris parmi les halogènes, les groupes trifluorométhyle, nitro, phénoxy, benzyloxy, alkyle ou alcoxy en $C_1$-$C_4$ ; ou bien un groupe cycloalkyle mono-, di-, tri- ou tétracyclique en $C_5$-$C_{10}$, à l'exception, toutefois, du 3-(benzylthio)-propionoate de S-benzyle $Ph—CH_2—S(CH_2)_2—CO—S—CH_2—Ph$.

2. 3-Alkylthiopropionothioates de S-alkyle de formule générale I selon la revendication 1 dans laquelle

R et $R^1$, identiques ou différents, représentent chacun un groupe alkyle substitué à chaîne droite ou ramifiée en $C_1$-$C_6$, ce groupe alkyle étant substitué par des groupes amino, alkylamino, dialkylamino contenant 1 à 4 atomes de carbone dans chacun des groupes alkyle, par des groupes phényle, naphtyle, phénoxy ou hétéroaryle à 5 ou 6 chaînons contenant en tant qu'hétéroatome un atome d'oxygène, de soufre ou d'azote, les groupes phényle et phénoxy mentionnés pouvant eux-mêmes, le cas échéant, porter 1 ou 2 substituants identiques ou différents choisis parmi les halogènes, les groupes trifluorométhyle, nitro, benzyloxy, alkyle en $C_1$-$C_2$ ou alcoxy en $C_1$-$C_2$, ou un reste adamantyle.

3. Le 3-(adamantyl-1-thio)-propionothioate de S-(adamantyle-1).

4. Procédé de préparation des composés de formule générale (I) selon les revendications 1 à 3, caractérisé en ce que :

a) on fait réagir des dérivés de l'acide acrylique de formule générale (II)

$$CH_2=CH-\overset{O}{\overset{\|}{C}}-X' \qquad (II)$$

dans laquelle

X' représente un halogène ou un groupe hydroxy, avec le double de la quantité équivalente d'un mercaptan de formule générale (IIIa)

$$R—SH \qquad (IIIa)$$

en présence d'un catalyseur basique, à des températures de 0 à 40 °C, ou bien

b) on fait réagir des thioéthers de formule générale (IV)

$$RS-CH_2-CH_2-\overset{O}{\overset{\|}{C}}-X \qquad (IV)$$

dans laquelle

X représente un groupe hydroxy, un halogène, un groupe acyloxy ou un reste d'ester de formule OR' dans laquelle R' représente un groupe alkyle, aryle ou aralkyle éventuellement substitué, éventuellement en présence d'un agent de condensation, avec un mercaptan de formule générale

(IIIa) R—SH ou (IIIb) $R^1$—SH

à des températures de 0 à 40 °C, R et $R^1$ ayant les significations indiquées ci-dessus, mais étant différents l'un de l'autre.

5. Composés de formule générale (I) selon la revendication 1, pour l'utilisation en tant que médicaments.

6. Composés de formule générale (I)

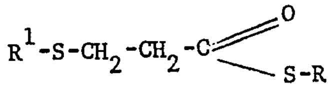

(I)

dans laquelle

R et $R^1$, identiques ou différents, représentent chacun un groupe alkyle substitué à chaîne droite ou ramifiée en $C_1$-$C_{10}$, le groupe alkyle étant substitué par des groupes amino, phényle, naphtyle, phénoxy, naphtoxy, alkylamino, dialkylamino contenant 1 à 4 atomes de carbone dans chacun des groupes alkyle ou par un groupe hétéroaryle à cycle de 5 à 7 chaînons et contenant en tant qu'hétéroatome un atome d'oxygène, de soufre ou d'azote, les groupes phényle, phénoxy et hétéroaryle mentionnés pouvant eux-mêmes, le cas échéant, porter 1, 2 ou 3 substituants identiques ou différents pris parmi les atomes d'halogènes, les groupes trifluorométhyle, nitro, phénoxy, benzyloxy, alkyle ou alcoxy contenant chacun 1 à 4 atomes de carbone, ou un groupe cycloalkyle mono-, di-, tri- ou tétra-cyclique en $C_5$-$C_{10}$, pour l'utilisation dans le traitement des processus inflammatoires.

7. Médicament contenant au moins un composé de formule générale (I) selon la revendication 1.

8. Médicament antiphlogistique contenant au moins un composé de formule générale (I)

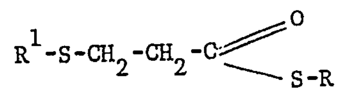

dans laquelle

R et $R^1$, identiques ou différents, représentent chacun un groupe alkyle substitué à chaîne droite ou ramifiée en $C_1$-$C_{10}$, le groupe alkyle étant substitué par des groupes amino, phényle, naphtyle, phénoxy, naphtoxy, alkylamino, dialkylamino contenant 1 à 4 atomes de carbone dans chacun des groupes alkyle, ou par un groupe hétéroaryle à cycle de 5 à 7 chaînons et contenant en tant qu'hétéroatome un atome d'oxygène, de soufre ou d'azote, les groupes phényle, phénoxy et hétéroaryle mentionnés pouvant eux-mêmes porter, le cas échéant, 1, 2 ou 3 substituants identiques ou différents pris parmi les halogènes, les groupes trifluorométhyle, nitro, phénoxy, benzyloxy, alkyle ou alcoxy contenant chacun 1 à 4 atomes de carbone, ou bien un reste cycloalkyle mono-, di-, tri- ou tétracyclique en $C_5$-$C_{10}$.

9. Procédé de préparation de médicaments, caractérisé en ce que l'on met des composés de formule générale (I) selon la revendication 1 sous une forme d'administration appropriée, le cas échéant en utilisant des produits auxiliaires et véhicules inertes.